(19) Europäisches Patentamt
European Patent Office
Office européen des brevets

(11) **EP 3 737 709 B1**

(12) **EUROPÄISCHE PATENTSCHRIFT**

(45) Veröffentlichungstag und Bekanntmachung des Hinweises auf die Patenterteilung:
**04.12.2024   Patentblatt 2024/49**

(21) Anmeldenummer: **19700006.0**

(22) Anmeldetag: **02.01.2019**

(51) Internationale Patentklassifikation (IPC):
**C08J 3/12** (2006.01)     **C08J 3/24** (2006.01)
**C08J 3/075** (2006.01)     **A61L 15/60** (2006.01)

(52) Gemeinsame Patentklassifikation (CPC):
**C08J 3/122; A61L 15/42; A61L 15/60; C08J 3/075; C08J 3/12; C08J 3/24; C08J 3/245;** C08J 2333/08; C08J 2433/08

(86) Internationale Anmeldenummer:
**PCT/EP2019/050010**

(87) Internationale Veröffentlichungsnummer:
**WO 2019/137833 (18.07.2019 Gazette 2019/29)**

(54) **SUPERABSORBERMISCHUNGEN**

SUPERABSORBER MIXTURES

MÉLANGES DE SUPERABSORBANTS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.01.2018   EP 18150837**

(43) Veröffentlichungstag der Anmeldung:
**18.11.2020   Patentblatt 2020/47**

(73) Patentinhaber: **BASF SE**
**67056 Ludwigshafen am Rhein (DE)**

(72) Erfinder:
• **BAUER, Stephan**
**67056 Ludwigshafen (DE)**
• **BAUMANN, Katrin**
**67056 Ludwigshafen (DE)**

• **TOENNESSEN, Markus**
**67056 Ludwigshafen (DE)**
• **BAUDUIN, Christophe**
**67056 Ludwigshafen (DE)**
• **BIEL, Markus Christian**
**52428 Jülich (DE)**
• **DANIEL, Thomas**
**67056 Ludwigshafen (DE)**

(74) Vertreter: **BASF IP Association**
**BASF SE**
**GBI - Z078**
**67056 Ludwigshafen (DE)**

(56) Entgegenhaltungen:
**WO-A1-2013/078109     WO-A1-2014/118024
WO-A1-2016/134905     WO-A1-2017/207330
DE-T2- 60 304 216**

**Beschreibung**

Die vorliegende Erfindung betrifft Superabsorbermischungen M, enthaltend mindestens 70 Gew.-% Superabsorber A mit einer Flüssigkeitsaufnahme von 20 g/g (T20) von weniger als 300 s und/oder einer volumetrischen Flüssigkeitsaufnahme unter 0,3 psi (2,07 kPa) Druck (VAUL) mit einem τ-Wert von weniger als 400 s und mindestens 5 Gew.-% Superabsorber B mit einer Zentrifugenretentionskapazität (CRC) von mindestens 30 g/g.

Superabsorber werden zur Herstellung von Windeln, Tampons, Damenbinden und anderen Hygieneartikeln, aber auch als wasserzurückhaltende Mittel im landwirtschaftlichen Gartenbau verwendet. Die Superabsorber werden auch als wasserabsorbierende Polymere bezeichnet.

Die Herstellung von Superabsorbern wird in der Monographie "Modern Superabsorbent Polymer Technology", F.L. Buchholz und A.T. Graham, Wiley-VCH, 1998, Seiten 71 bis 103, beschrieben.

Aufgabe der vorliegenden Erfindung war die Bereitstellung verbesserter Superabsorber, insbesondere von Superabsorbermischungen mit schneller Flüssigkeitsaufnahme und hoher Zentrifugen retentionskapazität.

Gelöst wurde die Aufgabe durch Superabsorbermischungen M, enthaltend mindestens 70 Gew.-% Superabsorber A mit einer Flüssigkeitsaufnahme von 20 g/g (T20) von weniger als 300 s und/oder einer volumetrischen Flüssigkeitsaufnahme unter 0,3 psi (2,07 kPa) Druck (VAUL) mit einem τ-Wert von weniger als 400 s und mindestens 5 Gew.-% Superabsorber B mit einer Zentrifugenretentionskapazität (CRC) von mindestens 30 g/g.

Die Flüssigkeitsaufnahme von 20 g/g (T20) des Superabsorbers A beträgt vorzugsweise weniger als 240 s, besonders bevorzugt weniger als 180 s, ganz besonders bevorzugt weniger als 120 s.

Bei der volumetrischen Flüssigkeitsaufnahme unter 0,3 psi (2,07 kPa) Druck (VAUL) beträgt der τ-Wert vorzugsweise weniger als 350 s, besonders bevorzugt weniger als 250 s, ganz besonders bevorzugt weniger als 200 s.

Die Flüssigkeitsaufnahme von 20 g/g (T20) und die volumetrischen Flüssigkeitsaufnahme unter 0,3 psi (2,07 kPa) Druck (VAUL) des Superabsorbers A kann durch Erhöhung der Menge an Vernetzer in der Monomerlösung verbessert werden. Es ist aber auch möglich das bei der Lösungspolymerisation erhaltene Polymergel vor der Trocknung zu extrudieren, wie in der WO 2014/118024 A1 beschrieben. Weiterhin ist es möglich die Menge an Oberflächennachvernetzer zu erhöhen.

Der Superabsorber A weist eine mittlere Sphärizität (mSPHT) von vorzugsweise weniger als 0,72, besonders bevorzugt von weniger als 0,70, ganz besonders bevorzugt von weniger als 0,68 auf.

Der Superabsorber A weist ein Schüttgewicht (ASG) von vorzugsweise weniger als 0,70 g/ml, besonders bevorzugt von weniger als 0,67 g/ml, ganz besonders bevorzugt von weniger als 0,64 g/ml, auf.

Der Superabsorber A wird beispielsweise durch Lösungspolymerisation hergestellt. Das dabei erhaltene Polymergel muss getrocknet und zerkleinert werden. Dies führt zu einer unregelmäßigen Form der Polymerpartikel und einem relativ niedrigen Schüttgewicht (ASG). Der Superabsorber A kann alternativ auch durch Vertropfungspolymerisation oder umgekehrte Suspensionspolymerisation hergestellt werden. Der Superabsorber A ist vorzugsweise oberflächennachvernetzt.

Die Zentrifugenretentionskapazität (CRC) des Superabsorbers B beträgt vorzugsweise mindestens 35 g/g, besonders bevorzugt mindestens 40 g/g, ganz besonders bevorzugt mindestens 45 g/g.

Die Zentrifugenretentionskapazität (CRC) des Superabsorbers B kann durch Senkung der Menge an Vernetzer in der Monomerlösung verbessert werden. Weiterhin ist es möglich die Menge an Oberflächennachvernetzer zu senken oder auf die Oberflächennachvernetzung zu verzichten.

Der Superabsorber B weist eine mittlere Sphärizität (mSPHT) von vorzugsweise größer 0,72, besonders bevorzugt von größer 0,76 ganz besonders bevorzugt von größer 0,80 auf.

Der Superabsorber B weist ein Schüttgewicht (ASG) von vorzugsweise größer 0,70 g/ml, besonders bevorzugt von größer 0,75 g/ml, ganz besonders bevorzugt von größer 0,80 g/ml, auf.

Der Superabsorber B wird beispielsweise durch Vertropfungspolymerisation hergestellt. Die dabei erhaltenen Polymerpartikel weisen eine weitgehend runde Form und ein relativ hohes Schüttgewicht (ASG) auf. Der Superabsorber B kann alternativ auch durch Lösungspolymerisation oder umgekehrte Suspensionspolymerisation hergestellt werden. Der Superabsorber B ist vorzugsweise nicht oberflächennachvernetzt.

Der Superabsorber A und/oder der Superabsorber B weist eine mittlere Partikelgröße von vorzugsweise 150 bis 850 μm, besonders bevorzugt von 200 bis 600 μm, ganz besonders bevorzugt von 250 bis 500 μm auf

Der vorliegenden Erfindung liegt die Erkenntnis zugrunde, dass sich die Flüssigkeitsaufnahme von 20 g/g (T20) und die volumetrischen Flüssigkeitsaufnahme unter 0,3 psi (2,07 kPa) Druck (VAUL) in Superabsorbermischungen nichtlinear verhält. Superabsorbermischungen, die überwiegend einen Superabsorber mit einer schnellen Flüssigkeitsaufnahme von 20 g/g (T20) und/oder einer schnellen volumetrischen Flüssigkeitsaufnahme unter 0,3 psi (2,07 kPa) Druck (VAUL) enthalten, weisen eine dem reinen Superabsorber vergleichbare Flüssigkeitsaufnahme von 20 g/g (T20) bzw. volumetrische Flüssigkeitsaufnahme unter 0,3 psi (2,07 kPa) Druck (VAUL) auf. Die Zentrifugenretentionskapazität (CRC) verhält sich dagegen in Superabsorbermischungen linear. Die Zentrifugenretentionskapazität (CRC) einer Superabsorbermischung kann daher mittels der Zentrifugenretentionskapazität (CRC) der reinen Superabsorber und deren Mischungsverhältnis berechnet werden.

Wird nun ein Superabsorber A mit einer schnellen Flüssigkeitsaufnahme von 20 g/g (T20) mit einem Superabsorber B mit einer hohen Zentrifugenretentionskapazität (CRC) gemischt, so erhält man eine Superabsorbermischung mit einer dem reinen Superabsorber A vergleichbaren Flüssigkeitsaufnahme von 20 g/g (T20) und einer gegenüber dem reinen Superabsorber A deutlich erhöhten Zentrifugenretentionskapazität (CRC).

Wird nun ein Superabsorber A mit einer schnellen volumetrischen Flüssigkeitsaufnahme unter 0,3 psi (2,07 kPa) Druck (VAUL) mit einem Superabsorber B mit einer hohen Zentrifugenretentionskapazität (CRC) gemischt, so erhält man eine Superabsorbermischung mit einer dem reinen Superabsorber A vergleichbaren volumetrischen Flüssigkeitsaufnahme unter 0,3 psi (2,07 kPa) Druck (VAUL) und einer gegenüber dem reinen Superabsorber A deutlich erhöhten Zentrifugenretentionskapazität (CRC).

Im Folgenden wird die Herstellung der Superabsorber näher erläutert:

Die Superabsorber können durch Polymerisation einer Monomerlösung oder -suspension, enthaltend

    a) mindestens ein ethylenisch ungesättigtes, säuregruppentragendes Monomer, das zumindest teilweise neutralisiert sein kann,
    b) mindestens einen Vernetzer und
    c) mindestens einen Initiator,

hergestellt werden und sind üblicherweise wasserunlöslich.

Die Monomeren a) sind vorzugsweise wasserlöslich, d.h. die Löslichkeit in Wasser bei 23° C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomere a) enthalten üblicherweise Polymerisationsinhibitoren, vorzugsweise Hydrochinonmonomethylether (MEHQ), als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonmonomethylether (MEHQ), jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonmonomethylether (MEHQ) verwendet werden.

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomers a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet. Weitere geeignete Vernetzer b) sind die in US 2017/0361305 beschriebenen "nano-clays", die in WO 00/31157 A1 beschrieben Wassergläser und die in WO 99/55767 A1 beschriebenen Aluminate.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 03/104299 A1, WO 03/104300 A1, WO 03/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 02/032962 A2 beschrieben.

Bevorzugte Vernetzer b) sind Pentaerythrittriallylether, Tetraallyloxyethan, Methylenbismethacrylamid, 15-fach ethoxyliertes Trimethylolpropantriacrylat, Polyethylenglykoldiacrylat, Trimethylolpropantriacrylat und Triallylamin.

Die Menge an Vernetzer b) beträgt vorzugsweise 0,25 bis 1,5 Gew.-%, besonders bevorzugt 0,3 bis 1,2 Gew.-%, ganz besonders bevorzugt 0,4 bis 0,8 Gew.-%, jeweils berechnet auf die Gesamtmenge an eingesetztem Monomer a). Mit steigendem Vernetzergehalt sinkt die Zentrifugenretentionskapazität (CRC) und die Absorption unter einem Druck von 21,0 g/cm$^2$ durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird vorzugsweise das Dinatriumsalz der 2-Hydroxy-2-sulfonatoessigsäure oder ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite® FF6 und Brüggolite® FF7 (Brüggemann Chemicals; Heilbronn; Deutschland) erhältlich. Es kann aber auch die reine 2-Hydroxy-2-sulfonatoessigsäure oder ein Salz davon als reduzierende Komponente eingesetzt werden, insbesondere wenn Ascorbinsäure mitverwendet wird.

In die Monomerlösung können vor oder während der Polymerisation noch Chelatbildner und 2-Hydroxycarbonsäuren zugesetzt werden wie beispielsweise in der WO 2017/170604 A1 beschrieben.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit die Löslichkeit überschreitendem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die Säuregruppen der erhaltenen Polymergele sind üblicherweise teilweise neutralisiert. Die Neutralisation wird auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 85 mol-%, besonders bevorzugt von 30 bis 80 mol-%, ganz besonders bevorzugt von 40 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Im Folgenden wird die Lösungspolymerisation erläutert:
Geeignete Reaktoren für die Lösungspolymerisation sind beispielsweise Knetreaktoren oder Bandreaktoren. Im Kneter wird das bei der Polymerisation einer wässrigen Monomerlösung oder -suspension entstehende Polymergel durch beispielsweise gegenläufige Rührwellen kontinuierlich zerkleinert, wie in WO 2001/038402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das zerkleinert werden muss, beispielsweise in einem Extruder oder Kneter.

Zur Verbesserung der Trocknungseigenschaften kann das mittels eines Kneters erhaltene zerkleinerte Polymergel zusätzlich extrudiert werden.

Das Polymergel wird dann in üblicherweise mit einem Umluftbandtrockner getrocknet bis der Restfeuchtegehalt vorzugsweise 0,5 bis 10 Gew.-%, besonders bevorzugt 1 bis 6 Gew.-%, ganz besonders bevorzugt 1,5 bis 4 Gew.-%, beträgt, wobei der Restfeuchtegehalt gemäß der von der EDANA empfohlenen Testmethode Nr. WSP 230.2-05 "Mass Loss Upon Heating" bestimmt wird. Bei einer zu hohen Restfeuchte weist das getrocknete Polymergel eine zu niedrige Glasübergangstemperatur $T_g$ auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Polymergels beträgt vor der Trocknung vorzugsweise von 25 und 90 Gew.-%, besonders bevorzugt von 35 bis 70 Gew.-%, ganz besonders bevorzugt von 40 bis 60 Gew.-%. Anschließend wird das getrocknete Polymergel gebrochen und optional grob zerkleinert.

Das getrocknete Polymergel wird hiernach üblicherweise gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion abgetrennten Polymerpartikel beträgt vorzugsweise von 150 bis 850 μm, besonders bevorzugt von 250 bis 600 μm, ganz besonders von 300 bis 500 μm. Die mittlere Partikelgröße der Produktfraktion kann mittels der von der EDANA empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Polymerpartikeln mit einer Partikelgröße von größer 150 μm beträgt vorzugsweise mindestens 90 Gew.-%, besonders bevorzugt mindestens 95 Gew.-%, ganz besonders bevorzugt mindestens 98 Gew.-%.

Polymerpartikel mit zu niedriger Partikelgröße senken die Gelbettpermeabilität (GBP). Daher sollte der Anteil zu kleiner Polymerpartikel ("fines") niedrig sein.

Zu kleine Polymerpartikel werden daher üblicherweise abgetrennt und in das Verfahren rückgeführt, vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung oder einem anderen Beschichtungsschritt. In diesem Fall sind die rückgeführten zu kleinen Polymerpartikel oberflächennachvernetzt bzw. anderweitig beschichtet, beispielsweise mit pyrogener Kieselsäure.

Wird zur Polymerisation ein Knetreaktor verwendet, so werden die zu kleinen Polymerpartikel vorzugsweise während des letzten Drittels der Polymerisation zugesetzt. Es ist aber auch möglich die zu kleinen Polymerpartikel in einem dem Polymerisationsreaktor nachgeschalteten Kneter oder Extruder in das Polymergel einzuarbeiten.

Werden die zu kleinen Polymerpartikel sehr früh zugesetzt, beispielsweise bereits zur Monomerlösung, so wird dadurch die Zentrifugenretentionskapazität (CRC) der erhaltenen Polymerpartikel gesenkt. Dies kann aber beispielsweise durch Anpassung der Einsatzmenge an Vernetzer b) kompensiert werden.

Der Anteil an Polymerpartikeln mit einer Partikelgröße von höchstens 850 $\mu$m, beträgt vorzugsweise mindestens 90 Gew.-%, besonders bevorzugt mindestens 95 Gew.-%, ganz besonders bevorzugt mindestens 98 Gew.-%.

Der Anteil an Polymerpartikeln mit einer Partikelgröße von höchstens 600 $\mu$m, beträgt vorzugsweise mindestens 90 Gew.-%, besonders bevorzugt mindestens 95 Gew.-%, ganz besonders bevorzugt mindestens 98 Gew.-%.

Polymerpartikel mit zu großer Partikelgröße senken die Quellgeschwindigkeit. Daher sollte der Anteil zu großer Polymerpartikel ebenfalls niedrig sein. Zu große Polymerpartikel werden daher üblicherweise abgetrennt und in die Mahlung rückgeführt.

Im Folgenden wird die Vertropfungspolymerisation erläutert:

Bei der Vertropfungspolymerisation wird die Monomerlösung mittels mindestens einer Bohrung unter Ausbildung von Tropfen in den Reaktor dosiert. Die Vertropfungspolymerisation wird beispielsweise in WO 2014/079694 A1 und WO 2015/110321 A1 beschrieben.

Die Bohrungen können sich beispielsweise in einer Vertropferplatte befinden. Die Anzahl und die Größe der Bohrungen werden gemäß der gewünschten Kapazität und Tropfengröße ausgewählt. Der Tropfendurchmesser beträgt dabei üblicherweise das 1,9-fache des Durchmessers der Bohrung. Wichtig ist hierbei, dass die zu vertropfende Flüssigkeit nicht zu schnell durch die Bohrung tritt bzw. der Druckverlust über die Bohrung nicht zu groß ist. Ansonsten wird die Flüssigkeit nicht vertropft, sondern der Flüssigkeitsstrahl wird infolge der hohen kinetischen Energie zerrissen (versprüht). Die Reynoldszahl bezogen auf den Durchsatz pro Bohrung und den Bohrungsdurchmesser ist vorzugsweise kleiner als 2.000, bevorzugt kleiner 1.600, besonders bevorzugt kleiner 1.400, ganz besonders bevorzugt kleiner 1.200.

Die Vertropferplatte weist vorzugsweise mindestens 5, besonders bevorzugt mindestens 25, ganz besonders bevorzugt mindestens 50, und vorzugsweise bis zu 750, besonders bevorzugt bis zu 500, ganz besonders bevorzugt bis zu 250, Bohrungen auf. Der Durchmesser der Bohrungen wird entsprechend der gewünschten Tropfengröße ausgewählt.

Der Durchmesser der Bohrungen beträgt vorzugsweise von 50 bis 500 $\mu$m, besonders bevorzugt von 70 bis 300 $\mu$m, ganz besonders bevorzugt von 100 bis 200 $\mu$m. Der Abstand der Bohrungen beträgt vorzugsweise das 50 bis 500 fache, besonders bevorzugt das 70 bis 300 fache, ganz besonders bevorzugt das 80 bis 200 fache des Bohrungsdurchmessers. Zu geringe Abstände führen zur Bildung von Agglomeraten, zu große Abstände verringern die Ausbeute.

Die Temperatur der Monomerlösung beim Durchtritt durch die Bohrungen beträgt vorzugsweise von 5 bis 80° C, besonders bevorzugt von 10 bis 70° C, ganz besonders bevorzugt von 30 bis 60° C.

Der Reaktor wird von einem Trägergas durchströmt. Dabei kann das Trägergas im Gleichstrom oder im Gegenstrom zu den frei fallenden Tropfen der Monomerlösung durch den Reaktor geführt werden, bevorzugt im Gleichstrom, d.h. von unten nach oben. Vorzugsweise wird das Trägergas nach einem Durchgang zumindest teilweise, bevorzugt zu mindestens 50%, besonders bevorzugt zu mindestens 75%, als Kreisgas in den Reaktor zurückgeführt. Üblicherweise wird eine Teilmenge des Trägergases nach jedem Durchgang ausgeschleust, vorzugsweise bis zu 10%, besonders bevorzugt bis zu 3%, ganz besonders bevorzugt bis zu 1%.

Der Sauerstoffgehalt des Trägergases beträgt vorzugsweise von 0,5 bis 15 Vol.-%, besonders bevorzugt von 1 bis 10 Vol.-%, ganz besonders bevorzugt von 2 bis 7 Vol.-%.

Das Trägergas enthält neben Sauerstoff vorzugsweise Stickstoff. Der Stickstoffgehalt des Trägergases beträgt vorzugsweise mindestens 80 Vol.-%, besonders bevorzugt mindesten 90 Vol.-%, ganz besonders bevorzugt mindestens 95 Vol.-%. Es können auch Gasmischungen verwendet werden. Das Trägergas kann auch mit Wasserdampf und/oder Acrylsäuredämpfen beladen werden.

Die Gasgeschwindigkeit wird vorzugsweise so eingestellt, dass die Strömung im Reaktor gerichtet ist, beispielsweise liegen keine der allgemeinen Strömungsrichtung entgegen gesetzte Konvektionswirbel vor, und beträgt üblicherweise 0,1 bis 2,5 m/s, vorzugsweise 0,3 bis 1,5 m/s bevorzugt von 0,5 bis 1,2 m/s, besonders bevorzugt 0,6 bis 1,0 m/s, ganz besonders bevorzugt 0,7 bis 0,9 m/s.

Das den Reaktor durchströmende Trägergas wird zweckmäßigerweise vor dem Reaktor auf die Reaktionstemperatur vorgewärmt.

Vorteilhaft wird die Gaseintrittstemperatur so geregelt, dass die Gasaustrittstemperatur, d.h. die Temperatur mit der das Trägergas den Reaktor verlässt üblicherweise von 90 bis 150° C, vorzugsweise von 100 bis 140° C, bevorzugt von 105 bis 135° C, besonders bevorzugt von 110 bis 130° C, ganz besonders bevorzugt von 115 bis 125° C, beträgt.

Die Reaktion kann im Überdruck oder im Unterdruck durchgeführt werden, ein Unterdruck von bis zu 100 mbar gegenüber dem Umgebungsdruck ist bevorzugt.

Das Reaktionsabgas, d.h. das den Reaktor verlassende Gas, kann beispielsweise in einem Wärmeaustauscher abgekühlt werden. Dabei kondensieren Wasser und nicht umgesetztes Monomer a). Danach kann das Reaktionsabgas zumindest teilweise wieder aufgewärmt und als Kreisgas in den Reaktor zurückgeführt werden. Ein Teil des Reaktionsabgases kann ausgeschleust und durch frisches Trägergas ersetzt werden, wobei im Reaktionsabgas enthaltenes Wasser und nicht umgesetzte Monomere a) abgetrennt und rückgeführt werden können.

Besonders bevorzugt ist ein Wärmeverbund, d.h., ein Teil der Abwärme beim Abkühlen des Abgases wird zum Aufwärmen des Kreisgases verwendet.

Die Reaktoren können begleitbeheizt werden. Die Begleitheizung wird dabei so eingestellt, dass die Wandtemperatur mindestens 5° C oberhalb der Reaktorinnentemperatur liegt und die Kondensation an den Reaktorwänden zuverlässig vermieden wird.

Im Folgenden wird die umgekehrte Suspensionspolymerisation beschrieben:
Bei der umgekehrten Suspensionspolymerisation wird die Monomerlösung während der Polymerisation in einem hydrophoben Lösungsmittel suspendiert. Die umgekehrte Suspensionspolymerisation wird beidpielsweise in WO 2008/068208 A1 und WO 2015/062883 A2 beschrieben.

Als hydrophobe Lösungsmittel sind alle dem Fachmann zum Einsatz bei der Suspensionspolymerisation bekannten Lösungsmittel einsetzbar. Bevorzugt werden aliphatische Kohlenwasserstoffe, wie n-Hexan, n-Heptan, n-Oktan, n-Nonan, n-Dekan, Cyclohexan oder Mischungen daraus, verwendet. Hydrophobe Lösungsmittel weisen bei 23° C eine Löslichkeit in Wasser zu weniger als 5 g/100 g, vorzugsweise weniger als 1 g/100 g, besonders bevorzugt weniger als 0,5 g/100 g, auf.

Das hydrophobe Lösungsmittel siedet im Bereich von vorzugsweise 50 bis 150° C, besonders bevorzugt 60 bis 120° C, ganz besonders bevorzugt 70 bis 90° C.

Das Verhältnis zwischen hydrophoben Lösungsmittel und Monomerlösung beträgt 0,5 bis 3, bevorzugt 0,7 bis 2,5 und ganz bevorzugt von 0,8 bis 2,2.

Der mittlere Durchmesser der Monomerlösungstropfen in der Suspension beträgt, wenn keine Agglomeration durchgeführt wird, vorzugsweise mindestens 100 $\mu$m, besonders bevorzugt von 100 bis 1000 $\mu$m, besonders bevorzugt von 150 bis 850 $\mu$m, ganz besonders bevorzugt von 300 bis 600 $\mu$m, wobei der Tropfendurchmesser durch Lichtstreuung bestimmt werden kann und den volumengemittelten mittleren Durchmesser bedeutet.

Der Durchmesser der Monomerlösungstropfen kann über die eingetragene Rührenergie und durch geeignete Dispergierhilfsmittel eingestellt werden.

Zur Dispergierung der wässrigen Monomerlösung im hydrophoben Lösungsmittel bzw. zur Dispergierung der entstehenden Superabsorberpartikel werden vorzugsweise Dispergierhilfsmittel zugesetzt. Es kann sich dabei um anionische, kationische, nichtionische oder amphotere Tenside, oder natürliche, halbsynthetische oder synthetische Polymere handeln.

Anionische Tenside sind beispielsweise Natriumpolyoxyethylendodecylethersulfat und Natriumdodecylethersulfat. Ein kationisches Tensid ist beispielsweise Trimethylstearylammoniumchlorid. Ein amphoteres Tensid ist beispielsweise Carboxymethyldimethylcetylammonium. Nichtionische Tenside sind beispielsweise Saccharosefettsäureester, wie Saccharosemonostearat und Saccharosedilaurat, Sorbitanester, wie Sorbitanmonostearat, Polyoxyalkylen-Verbindungen auf Basis von Sorbitanestern, wie Polyoxyethylensorbitanmonostearat.

Das Dispergierhilfsmittel wird üblicherweise im hydrophoben Lösungsmittel gelöst oder dispergiert. Das Dispergiermittel wird in Mengen zwischen 0.01 und 10 Gew.-%, bevorzugt zwischen 0,2 und 5 Gew.-%, besonders bevorzugt zwischen 0,5 und 2 Gew.-%, bezogen auf die Monomerlösung, eingesetzt. Über Art und Menge des Dispergierhilfsmittels kann der Durchmesser der Monomerlösungstropfen eingestellt werden.

Vorteilhaft werden für die Polymerisation mehrere Rührreaktoren hintereinander geschaltet. Durch die Nachreaktion in weiteren Rührreaktoren kann der Monomerumsatz erhöht und die Rückvermischung vermindert werden. Hierbei ist es weiterhin vorteilhaft, wenn der erste Rührreaktor nicht zu groß ist. Mit steigender Größe des Rührreaktors verbreitert sich zwangsläufig die Größenverteilung der dispergierten Monomerlösungstropfen. Ein kleinerer erster Reaktor ermöglicht daher die Herstellung Superabsorberpartikel mit besonders enger Partikelgrößenverteilung.

Die Reaktion wird vorzugsweise unter vermindertem Druck durchgeführt, beispielsweise bei einem Druck von 800 mbar. Über den Druck kann der Siedepunkt der Reaktionsmischung auf die gewünschte Reaktionstemperatur eingestellt werden.

Wird die Polymerisation unter ausreichendem Rückfluss durchgeführt, so kann auf die Inertisierung verzichtet werden. Dabei wird der gelöste Sauerstoff zusammen mit dem verdampfenden Lösungsmittel aus dem Polymerisationsreaktor entfernt.

Die Superabsorberpartikel können in der Polymerdispersion azeotrop entwässert, aus der Polymerdispersion abgetrennt und die abgetrennten Superabsorberpartikel können zur Entfernung des anhaftenden restlichen hydrophoben Lösungsmittels getrocknet werden.

Im Folgenden wird die Oberflächennachvernetzung beschrieben:

Die Polymerpartikel können zur weiteren Verbesserung der Eigenschaften thermisch oberflächennachvernetzt werden. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, $\beta$-Hydroxyalkylamide, wie in DE 102 04 938 A1 und US 6,239,230 beschrieben, oder Oxazoline, wie in EP 0 999 938 A2 beschrieben.

Des Weiteren sind in DE 40 20 780 C1 zyklische Karbonate, in DE 198 07 502 A1 2-Oxazolidinon und dessen Derivate, wie 2-Hydroxyethyl-2-oxazolidinon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidinone, in DE 102 04 937 A1 zyklische Harnstoffe, in EP 2 204 388 A1 Oxetane, in DE 103 34 584 A1 bizyklische Amidoacetale, in EP 1 199 327 A2 Oxetane und zyklische Harnstoffe und in WO 03/031482 A1 Morpholin-2,3-dion und dessen Derivate als geeignete Oberflächennachvernetzer beschrieben.

Bevorzugte Oberflächennachvernetzer sind Ethylenkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer sind 2-Hydroxyethyl-2-oxazolidinon, 2-Oxazolidinon und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben

Die Menge an Oberflächennachvernetzer beträgt vorzugsweise 0,001 bis 3 Gew.-%, besonders bevorzugt 0,02 bis 1 Gew.-%, ganz besonders bevorzugt 0,05 bis 0,2 Gew.-%, jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel oberflächennachvernetzt und getrocknet, wobei die Oberflächennachvernetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; Deutschland), Vrieco-Nauta Continuous Mixer (Hosokawa Micron BV; Doetinchem; Niederlande), Processall Mixmill Mixer (Processall Incorporated; Cincinnati; USA) und Schugi Flexomix® (Hosokawa Micron BV; Doetinchem; Niederlande). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser, Propylenglykol/Wasser, 2-Methyl-1,3-propandiol/Wasser, Ethylenglykol/Wasser, Diethylenglykol/Wasser, Triethylenglykol/Wasser, Tetraethylenglykol/Wasser, oder Polyethylenglykol/Wasser, wobei das Mischungsmassenverhältnis vorzugsweise von 20:80 bis 40:60 beträgt.

Die Oberflächennachvernetzung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; Deutschland), Hosokawa Bepex® Disc Dryer (Hosokawa Micron GmbH; Leingarten; Deutschland), Holo-Flite® dryers (Metso Minerals Industries Inc.; Danville; USA) und Nara Paddle Dryer (NARA Machinery Europe; Frechen; Deutschland). Überdies können auch Wirbelschichttrockner eingesetzt werden.

Die Oberflächennachvernetzung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und thermisch oberflächen nachvernetzt.

Bevorzugte Reaktionstemperaturen liegen im Bereich 100 bis 250° C, bevorzugt 120 bis 220° C, besonders bevorzugt 130 bis 210° C, ganz besonders bevorzugt 150 bis 200° C. Die bevorzugte Verweilzeit bei dieser Temperatur beträgt vorzugsweise mindestens 10 Minuten, besonders bevorzugt mindestens 20 Minuten, ganz besonders bevorzugt mindestens 30 Minuten, und üblicherweise höchstens 60 Minuten.

In einer bevorzugten Ausführungsform der vorliegenden Erfindung werden die Polymerpartikel nach der Oberflächennachvernetzung gekühlt. Die Kühlung wird vorzugsweise in Kontaktkühlern, besonders bevorzugt Schaufelkühlern, ganz besonders bevorzugt Scheibenkühlern, durchgeführt. Geeignete Kühler sind beispielsweise Hosokawa Bepex® Horizontal Paddle Cooler (Hosokawa Micron GmbH; Leingarten; Deutschland), Hosokawa Bepex® Disc Cooler (Hosokawa Micron GmbH; Leingarten; Deutschland), Holo-Flite® coolers (Metso Minerals Industries Inc.; Danville; USA) und Nara Paddle Cooler (NARA Machinery Europe; Frechen; Deutschland). Überdies können auch Wirbelschichtkühler eingesetzt werden.

Im Kühler werden die Polymerpartikel auf vorzugsweise 40 bis 90° C, besonders bevorzugt 45 bis 80° C, ganz besonders bevorzugt 50 bis 70° C, abgekühlt.

Die Polymerpartikel können zur weiteren Verbesserung der Eigenschaften beschichtet oder nachbefeuchtet werden:

Die Nachbefeuchtung wird vorzugsweise bei 40 bis 120° C, besonders bevorzugt bei 50 bis 110° C, ganz besonders bevorzugt bei 60 bis 100° C, durchgeführt. Bei zu niedrigen Temperaturen neigen die Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die Nachbefeuchtung wird die mechanische Stabilität der Polymerpartikel erhöht und deren Neigung zur statischen Aufladung vermindert.

Geeignete Beschichtungen zur Verbesserung der Quellgeschwindigkeit sowie der Gelbettpermeabilität (GBP) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span® 20. Geeignete Beschichtungen zur Staubbindung, zur Verringerung der Verbackungsneigung sowie zur Erhöhung der mechanischen Stabilität sind Polymerdispersionen, wie in EP 0 703 265 B1 beschrieben, und Wachse, wie in US 5 840 321 beschrieben.

Methoden:

Die nachfolgend beschriebenen, mit "WSP" bezeichneten Standard-Testmethoden werden beschrieben in: "Standard Test Methods for the Nonwovens Industry", Ausgabe 2005, gemeinsam herausgegeben von den "Worldwide Strategie Partners" EDANA (Avenue Eugène Plasky, 157, 1030 Brüssel, Belgien, www.edana.org) und INDA (1100 Crescent Green, Suite 115, Cary, North Carolina 27518, USA, www.inda.org). Diese Veröffentlichung ist sowohl von EDANA als auch von INDA erhältlich.

Die Messungen sollten, wenn nicht anders angegeben, bei einer Umgebungstemperatur von 23 ± 2 ° C und einer relativen Luftfeuchte von 50 ± 10 % durchgeführt werden. Die Superabsorberpartikel werden vor der Messung gut durchmischt.

Flüssigkeitsaufnahme von 20 g/g (T20)

Die Flüssigkeitsaufnahme von 20 g/g (T20) wird gemäß der in der EP 2 535 027 A1 auf den Seiten 13 bis 18 beschriebenen Testmethode "K(t) Test Method (Dynamic Effective Permeability and Uptake Kinetics Measurement Test Method)" bestimmt.

volumetrische Flüssigkeitsaufnahme unter 0,3 psi (2,07 kPa) Druck (VAUL)

Bei der volumetrischen Flüssigkeitsaufnahme unter 0,3 psi (2,07 kPa) Druck (VAUL) wird der $\tau$-Wert gemäß der in der WO 2014/079694 A1 auf den Seiten 39 und 40 beschriebenen Testmethode "Volumetric Absorbency Under Load (VAUL)" bestimmt. Der $\tau$-Wert wird dort als "characteristic swelling time" bezeichnet.

Restmonomer

Der Gehalt an Restmonomer wird gemäß der von der EDANA empfohlenen Testmethode WSP Nr. 210.2 (05) "Residual Monomers" bestimmt.

Feuchtegehalt

Der Feuchtegehalt wird gemäß der von der EDANA empfohlenen Testmethode Nr. WSP 230.2 (05) "Mass Loss Upon Heating" bestimmt.

### Quellkapazität (Free Swell Capacity)

Die Quellkapazität (FSC) wird gemäß der von der EDANA empfohlenen Testmethode Nr. WSP 240.2 (05) "Free Swell Capacity in Saline, Gravimetric Determination" bestimmt.

### Zentrifugenretentionskapazität (Centrifuge Retention Capacity)

Die Zentrifugenretentionskapazität (CRC) wird gemäß der von der EDANA empfohlenen Testmethode Nr. WSP 241.2 (05) "Fluid Retention Capacity in Saline, After Centrifugation" bestimmt.

### Absorption unter einem Druck von 0,0 g/cm$^2$ (Absorption under No Load)

Die Absorption unter einem Druck von 0,0 g/cm$^2$ (AUNL) wird analog der von der EDANA empfohlenen Testmethode Nr. WSP 242.2 (05) "Absorption Under Pressure, Gravimetric Determination" bestimmt, wobei statt eines Drucks von 21,0 g/cm$^2$ (0.3psi) ein Druck von 0,0 g/cm$^2$ (0.0psi) eingestellt wird.

### Absorption unter einem Druck von 21,0 g/cm$^2$ (Absorption under Load)

Die Absorption unter einem Druck von 21,0 g/cm$^2$ (AUL) wird gemäß der von der EDANA empfohlenen Testmethode Nr. WSP 242.2 (05) "Absorption Under Pressure, Gravimetric Determination" bestimmt.

### Absorption unter einem Druck von 49,2 g/cm$^2$ (Absorption under High Load)

Die Absorption unter einem Druck von 49,2 g/cm$^2$ (AUHL) wird analog der von der EDANA empfohlenen Testmethode Nr. WSP 242.2 (05) "Absorption Under Pressure, Gravimetric Determination" bestimmt, wobei statt eines Drucks von 21,0 g/cm$^2$ (0.3psi) ein Druck von 49,2 g/cm$^2$ (0.7psi) eingestellt wird.

### Extrahierbare

Der Gehalt an extrahierbaren Bestandteilen der Superabsorberpartikel wird gemäß der von der EDANA empfohlenen Testmethode Nr. WSP 270.2 (05) "Extractable" bestimmt.

### Flüssigkeitsweiterleitung (Saline Flow Conductivity)

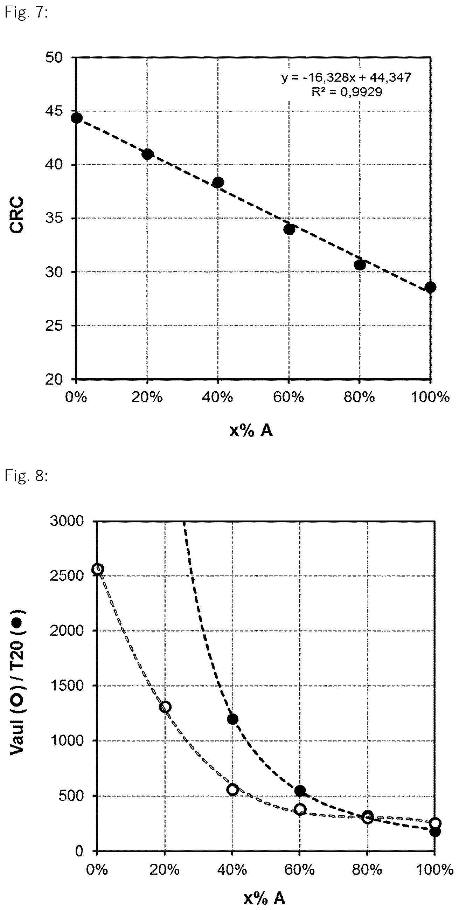

Die Flüssigkeitsweiterleitung (SFC) einer gequollenen Gelschicht unter Druckbelastung von 0,3 psi (2070 Pa) wird, wie in EP 0 640 330 A1 beschrieben, als Gel-Layer-Permeability einer gequollenen Gelschicht aus Superabsorberpartikeln bestimmt, wobei die in zuvor genannter Patentanmeldung auf Seite 19 und in Figur 8 beschriebene Apparatur dahingehend modifiziert wurde, dass die Glasfritte (40) nicht mehr verwendet wird, der Stempel (39) aus gleichem Kunststoffmaterial besteht wie der Zylinder (37) und jetzt über die gesamte Auflagefläche gleichmäßig verteilt 21 gleichgroße Bohrungen enthält. Die Vorgehensweise sowie Auswertung der Messung bleibt unverändert gegenüber EP 0 640 330 A1. Der Durchfluss wird automatisch erfasst.

Die Flüssigkeitsweiterleitung (SFC) wird wie folgt berechnet:

$$SFC \ [cm^3s/g] = (Fg(t=0) \ x \ L_0)/(d \ x \ A \ x \ WP),$$

wobei Fg(t=0) der Durchfluss an NaCl-Lösung in g/s ist, der anhand einer linearen Regressionsanalyse der Daten Fg(t) der Durchflussbestimmungen durch Extrapolation gegen t=0 erhalten wird, $L_0$ die Dicke der Gelschicht in cm, d die Dichte der NaCl-Lösung in g/cm$^3$, A die Fläche der Gelschicht in cm$^2$ und WP der hydrostatische Druck über der Gelschicht in dyn/cm$^2$.

### Vortex-Test

In ein 100 ml-Becherglas, welches ein Magnetrührstäbchen der Größe 30 mm x 6 mm enthält, werden 50,0 ml $\pm$ 1,0 ml einer 0,9 Gew.-%igen wässrigen Kochsalzlösung gegeben. Mit Hilfe eines Magnetrührers wird die Kochsalzlösung bei 600 Upm gerührt. Es werden dann möglichst schnell 2,000 g $\pm$ 0,010 g Superabsorberpartikel zugegeben, und die Zeit gemessen, die vergeht, bis die Rührtraube durch die Absorption der Kochsalzlösung durch die Superabsorberpartikel verschwindet. Dabei kann der ganze Inhalt des Becherglases sich als einheitliche Gelmasse immer noch drehen, aber die Oberfläche der gelierten Kochsalzlösung darf keine individuellen Turbulenzen mehr zeigen. Die benötigte Zeit wird als Vortex berichtet.

mittlere Sphärizität (mSPHT)

Die mittlere Sphärizität (mSPHT) wird mit dem PartAn® 3001 L Partikel Analysator (Microtrac Europe GmbH; Deutschland) bestimmt.

Die zu analysierende Probe wird in einen Trichter eingefüllt. Das rechnergesteuerte Messsystem startet die Dosiervorrichtung und sorgt für einen kontinuierlichen, konzentrationsgeregelten Partikelstrom. Die Partikel fallen vereinzelt durch den Messschacht und erzeugen kontrastreiche Schattenbilder zwischen Lichtquelle und hochauflösender Kamera. Die Lichtquelle wird von der Kamera angesteuert und erzeugt aufgrund sehr kurzer Belichtungszeiten fehlerfreie Bildinformationen für die Mehrfach-Auswertung jedes einzelnen Partikels in Echtzeit.

In einem 3D-Verfahren wird jedes Partikel mehrfach analysiert und das Verfahren liefert so die absoluten Ergebnisse zur Länge, Breite, Dicke, Fläche und Umfang. Über die Anzahl der vom Partikel abgedeckten Pixel wird die Größe und Form berechnet.

Schüttgewicht

Das Schüttgewicht (ASG) wird gemäß der von der EDANA empfohlenen Testmethode Nr. WSP 260.2 (05) "Density, Gravimetric Determination" bestimmt.

mittlere Partikelgröße

Die mittlere Partikelgröße wird mittels der von der EDANA empfohlenen Testmethode Nr. WSP 220.2 (05) "Partikel Size Distribution" ermittelt, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Beispiele

Herstellungsbeispiel 1 - Superabsorber B

Es wurde ein Grundpolymer analog zu Beispiel 1 der WO 2017/207330 A1 hergestellt. Der Neutralisationsgrad betug 72,0 mol-% und der Feststoffgehalt der Monomerlösung betrug 43,0 Gew.-%.

Als mehrfach ungesättigter Vernetzer wurde 3-fach ethoxiliertes Glyzerintriacrylat eingesetzt. Der Vernetzer wurde gemäß Beispiel 7 der WO 03/104299 A1 hergestellt. Der Vernetzer wurde in einer Menge von 0,20 Gew.-% eingesetzt, bezogen auf Acrylsäure vor der Neutralisation. Zusätzlich enthielt die Monomerlösung 0,75 Gew.-% Polyethylenglykol-4000 (Polyethylenglykol mit einem mittleren Molgewicht von 4000 g/mol), wieder bezogen auf Acrylsäure vor der Neutralisation.

Als Polymerisationsinitiatoren wurden 0,16 Gew.-% Natriumperoxodisulfat, 0,0007 Gew.-% Wasserstoffperoxid und 0,0028 Gew.-% Ascorbinsäure eingesetzt, jeweils bezogen auf Acrylsäure vor der Neutralisation.

Der hergestellte Superabsorber (Grundpolymer) hatte eine Zentrifugenretentionskapazität (CRC) von 44,8 g/g, eine Absorption unter einem Druck von 0,0 g/cm$^2$ (AUNL) von 48,2 g/g, eine Absorption unter einem Druck von 21,0 g/cm$^2$ (AUL) von 7,8 g/g, eine Quellkapazität (FSC) von 56,9 g/g, einen Feuchtegehalt von 3,2 Gew.-% und ein Schüttgewicht (ASG) von 0,67 g/ml.

Der Superabsorber wies die folgende Partikelgrößenverteilung auf:

| | |
|---|---|
| >710 $\mu$m | 0,2 Gew.-% |
| 600 - 710 $\mu$m | 14,3 Gew.-% |
| 500 - 600 $\mu$m | 19,0 Gew.-% |
| 400 - 500 $\mu$m | 28,8 Gew.-% |
| 300 - 400 $\mu$m | 19,0 Gew.-% |
| 200 - 300 $\mu$m | 12,3 Gew.-% |
| 150 - 200 $\mu$m | 5,2 Gew.-% |
| 106 - 150 $\mu$m | 1,2 Gew.-% |

(fortgesetzt)

| | |
|---|---|
| <106 μm | 0,0 Gew.-% |

Der Superabsorber wies eine mittlere Partikelgröße (d50) von 430 μm und eine mittelere Sphärizität (mSPHT) von 0,60 auf.

Herstellungsbeispiel 2 - Superabsorber B

1200 g des Grundpolymers aus Herstellbeispiel 1 wurden in einem Pflugschar®-Mischer mit Heizmantel vom Typ M5 (Gebr. Lödige Maschienbau GmbH, Paderborn, Deutschland) vorgelegt und bei 23° C und einer Wellendrehzahl von 200 Umdrehungen pro Minute mittels einer Zweistoff-Sprühdüse mit folgender Lösung beschichtet, jeweils bezogen auf das Grundpolymer:

0,04 Gew.-% Ethylenglykoldiglycidylether
1,5 Gew.-% Propylenglykol
0,05 Gew.-% Aluminiumsulfat
3,0 Gew.-% Wasser

Die Wellendrehzahl wurde auf 60 Umdrehungen pro Minute eingestellt und das Produkt auf eine Temperatur von 120° C gebracht. Um diese Temperatur zu halten, wurde die Temperatur der Heizflüssigkeit passend reduziert. Nach 30 Minuten bei 120° C wurde auf Umgebungstemperatur abgekühlt.
Anschließend wurde die erhaltenen Polymerpartikel in einem weiteren Mischer bei 80° C rückbefeuchtet. Hierzu wurde 7,0 Gew.-% einer 0,05% Gew.-%igen wässrigen Aluminiumsulfatlösung mittels einer mit Stickstoff betriebenen Zweistoffdüse und unter Rühren auf das Polymer aufgesprüht. Unter Rühren wurde das Polymer innerhalb von 20 Minuten auf 25° C abgekühlt und auf eine Partikelgröße von 150 bis 710 μm abgesiebt.
Der hergestellte Superabsorber hatte eine Zentrifugenretentionskapazität (CRC) von 33,4 g/g, eine Absorption unter einem Druck von 0,0 g/cm$^2$ (AUNL) von 48,3 g/g, eine Absorption unter einem Druck von 21,0 g/cm$^2$ (AUL) von 31,7 g/g, eine Absorption unter einem Druck von 49,2 g/cm$^2$ (AUHL) von 23,9 g/g, eine Quellkapazität (FSC) von 54,4 g/g, einen Feuchtegehalt von 5,0 Gew.-%, eine Flüssigkeitsweiterleitung (SFC) von 8 x 10$^{-7}$ cm$^3$s/g, einen Vortex von 52 s, einen Gehalt an Restmonomer von 310 ppm und ein Schüttgewicht (ASG) von 0,67 g/ml.
Der Superabsorber wies die folgende Partikelgrößenverteilung auf:

| | |
|---|---|
| > 710 μm | 0,50 Gew.-% |
| 600 - 710 um | 11,2 Gew.-% |
| 500 - 600 μm | 20,9 Gew.-% |
| 425 - 500 μm | 24,5 Gew.-% |
| 300 - 425 μm | 23,6 Gew.-% |
| 150 - 300 μm | 18,0 Gew.-% |
| 106 - 150 μm | 0,4 Gew.-% |
| <106 μm | 0,0 Gew.-% |

Der Superabsorber wies eine mittlere Partikelgröße (d50) von 464 μm und eine mittlere Sphärizität (mSPHT) von 0,60 auf.

Herstellungsbeispiel 3 - Superabsorber A

Es wurde ein Superabsorber gemäß Beispiel 14 der WO 2014/118024 A1 hergestellt. Der hergestellte Superabsorber hatte ein Schüttgewicht (ASG) von 0,60 g/ml, eine Zentrifugenretentionskapazität (CRC) von 26,0 g/g, eine Absorption unter einem Druck von 49,2 g/cm$^2$ (AUHL) von 23,9 g/g und eine Flüssigkeitsweiterleitung (SFC) von 136 x 10$^{-7}$ cm$^3$s/g.
Der Superabsorber wies die folgende Partikelgrößenverteilung auf:

| | |
|---|---|
| >710 μm | 8,7 Gew.-% |
| 600 - 710 μm | 19,2 Gew.-% |
| 500 - 600 μm | 26,9 Gew.-% |

(fortgesetzt)

| | |
|---|---|
| 400 - 500 μm | 21,9 Gew.-% |
| 300 - 400 μm | 14,2 Gew.-% |
| 150 - 300 μm | 9,0 Gew.-% |
| 106 - 150 um | 0,1 Gew.-% |
| <106 μm | 0,0 Gew.-% |

Der Superabsorber wies eine mittlere Partikelgröße (d50) von 510 μm und eine mittlere Sphärizität (mSPHT) von 0,66 auf.

Herstellungsbeispiel 4 - Superabsorber B

Es wurde ein Superabsorber analog zu Example 2 der WO 2016/134905 A1 hergestellt. Die eingesetzte Monomerlösung enthielt zusätzlich 1,07 Gew.-% des Dinatriumsalzes der 1-Hydroxyethyliden-1,1-diphosphonsäure.
Die Gaseingangstemperatur der Reaktionszone (5) betrug 167° C, die Gasausgangstemperatur der Reaktionszone (5) betrug 107° C, die Gaseingangstemperatur des interen Wirbelbetts (27) betrug 100° C, die Produkttemperatur im internen Wirbelbett (27) betrug 78° C, die Gasausgangstemperatur der Kondensationskolonne (12) betrug 57° C und die Gasausgangstemperatur der Gastrocknungseinheit (37) betrug 47° C.
Der hergestellte Superabsorber (Grundpolymer) hatte ein Schüttgewicht (ASG) von 0,681 g/ml, eine Zentrifugenretentionskapazität (CRC) von 56,8 g/g, eine Absorption unter einem Druck von 21,0 $g/cm^2$ (AUL) von 8,1 g/g, einen Gehalt an Restmonomer von 8550 ppm, einen Gehalt an Extrahierbaren von 9,7 Gew.-% und einen Feuchtegehalt von 8,5 Gew.-%.
Der Superabsorber wies die folgende Partikelgrößenverteilung auf:

| | |
|---|---|
| >1000 μm | 0,3 Gew.-% |
| 850 - 1000 μm | 1,1 Gew.-% |
| 600 - 850 μm | 4,7 Gew.-% |
| 500 - 600 μm | 13,9 Gew.-% |
| 400 - 500 μm | 35,5 Gew.-% |
| 300 - 400 μm | 34,3 Gew.-% |
| 250 - 300 μm | 6,6 Gew.-% |
| 200 - 250 μm | 3,1 Gew.-% |
| 106 - 200 μm | 0,4 Gew.-% |
| <106 μm | 0,1 Gew.-% |

Der Superabsorber wies eine mittlere Partikelgröße (d50) von 397 μm und eine mittlere Sphärizität (mSPHT) von 0,81 auf.
Das Grundpolymer wurde anschließend analog zu den Beispielen 11 bis 15 der WO 2015/110321 A1 oberflächennachvernetzt. Es wurden 2,0 Gew.-% Ethylenkarbonat, 5,0 Gew.-% Wasser und 0,4 Gew.-% Aluminiumsulfat eingesetzt, jeweils bezogen auf das Grundpolymer. Die Produkttemperatur betrug 150° C und die Höhe des Wehrs betrug 75%.
Im Kühler nach der Oberflächennachbvernetzung wurden 4,7 Gew.-% einer 0,1 gew.-%igen wässrigen Lösung von Sorbitanmonolaurat zugesetzt.
Der hergestellte Superabsorber hatte ein Schüttgewicht (ASG) von 0,753 g/ml, eine Zentrifugenretentionskapazität (CRC) von 46,8 g/g, eine Absorption unter einem Druck von 21,0 $g/cm^2$ (AUL) von 33,0 g/g, eine Absorption unter einem Druck von 49,2 $g/cm^2$ (AUHL) von 16,7 g/g, eine Flüssigkeitsweiterleitung (SFC) von 0 x $10^{-7}$ $cm^3s/g$, einen Vortex von 60 s, einen Feuchtegehalt von 4,4 Gew.-%, einen Gehalt an Restmonomer von 460 ppm und einen Gehalt an Extrahierbaren von 5,0 Gew.-%.
Der oberflächennachvernetzte Superabsorber wies die folgende Partikelgrößenverteilung auf:

| | |
|---|---|
| >850 μm | 0,0 Gew.-% |
| 710 - 850 μm | 0,6 Gew.-% |
| 600 - 710 μm | 4,5 Gew.-% |
| 500 - 600 μm | 10,9 Gew.-% |

(fortgesetzt)

| | |
|---|---|
| 400 - 500 μm | 40,6 Gew.-% |
| 300 - 400 μm | 34,0 Gew.-% |
| 250 - 300 μm | 6,1 Gew.-% |
| 200 - 250 μm | 2,6 Gew.-% |
| 150 - 200 μm | 0,5 Gew.-% |
| <150 μm | 0,2 Gew.-% |

Der Superabsorber wies eine mittlere Partikelgröße (d50) von 405 μm und eine mittlere Sphärizität (mSPHT) 0,80 auf.

Herstellungsbeispiel 5 - Superabsorber A

Es wurde ein Superabsorber analog zu Example 2 der WO 2016/134905 A1 hergestellt. Die eingesetzte Monomerlösung enthielt zusätzlich 1,07 Gew.-% des Dinatriumsalzes der 1-Hydroxyethyliden-1,1-diphosphonsäure. Außerdem wurde eine Vertropferplatte mit 645 Bohrungen, einem Bohrungsdurchmesser von 100 μm und einem Bohrungsabstand von 8 mm eingesetzt.

Die Gaseingangstemperatur der Reaktionszone (5) betrug 167° C, die Gasausgangstemperatur der Reaktionszone (5) betrug 108° C, die Gaseingangstemperatur des interen Wirbelbetts (27) betrug 94° C, die Produkttemperatur im internen Wirbelbett (27) betrug 73° C, die Gasausgangstemperatur der Kondensationskolonne (12) betrug 58° C und die Gasausgangstemperatur der Gastrocknungseinheit (37) betrug 47° C.

Der hergestellte Superabsorber (Grundpolymer) hatte ein Schüttgewicht (ASG) von 0,648 g/ml, eine Zentrifugenretentionskapazität (CRC) von 54,7 g/g, eine Absorption unter einem Druck von 21,0 g/cm$^2$ (AUL) von 8,8 g/g, einen Gehalt an Restmonomer von 12600 ppm, einen Gehalt an Extrahierbaren von 8,9 Gew.-% und einen Feuchtegehalt von 9,0 Gew.-%.

Der Superabsorber wies die folgende Partikelgrößenverteilung auf:

| | |
|---|---|
| >1000 μm | 0,1 Gew.-% |
| 850 - 1000 μm | 0,1 Gew.-% |
| 600 - 850 μm | 0,6 Gew.-% |
| 500 - 600 μm | 2,6 Gew.-% |
| 400 - 500 μm | 13,1 Gew.-% |
| 300 - 400 μm | 41,5 Gew.-% |
| 250 - 300 μm | 22,0 Gew.-% |
| 200 - 250 μm | 15,0 Gew.-% |
| 106 - 200 μm | 4,9 Gew.-% |
| <106 μm | 0,1 Gew.-% |

Der Superabsorber wies eine mittlere Partikelgröße (d50) von 284 μm und eine mittlere Sphärizität (mSPHT) von 0,82 auf.

Das Grundpolymer wurde anschließend analog zu den Beispielen 11 bis 15 der WO 2015/110321 A1 oberflächennachvernetzt. Es wurden 2,0 Gew.-% Ethylenkarbonat, 5,0 Gew.-% Wasser und 0,4 Gew.-% Aluminiumsulfat eingesetzt, jeweils bezogen auf das Grundpolymer. Die Produkttemperatur betrug 175° C und die Höhe des Wehrs betrug 75%.

Im Kühler nach der Oberflächennachbvernetzung wurden 4,7 Gew.-% einer 0,1 gew.-%igen wässrigen Lösung von Sorbitanmonolaurat zugesetzt.

Der hergestellte Superabsorber hatte ein Schüttgewicht (ASG) von 0,733 g/ml, eine Zentrifugenretentionskapazität (CRC) von 28,6 g/g, eine Absorption unter einem Druck von 21,0 g/cm$^2$ (AUL) von 29,2 g/g, eine Absorption unter einem Druck von 49,2 g/cm$^2$ (AUHL) von 23,8 g/g, eine Flüssigkeitsweiterleitung (SFC) von $32 \times 10^{-7}$ cm$^3$s/g, einen Vortex von 43 s, einen Feuchtegehalt von 3,5 Gew.-%, einen Gehalt an Restmonomer von 790 ppm und einen Gehalt an Extrahierbaren von 1,5 Gew.-%.

Der oberflächennachvernetzte Superabsorber wies die folgende Partikelgrößenverteilung auf:

| | |
|---|---|
| >850 μm | 0,2 Gew.-% |
| 710 - 850 μm | 0,2 Gew.-% |

(fortgesetzt)

| | |
|---|---|
| 600 - 710 µm | 0,9 Gew.-% |
| 500 - 600 µm | 2,1 Gew.-% |
| 400 - 500 µm | 13,5 Gew.-% |
| 300 - 400 µm | 41,9 Gew.-% |
| 250 - 300 µm | 24,2 Gew.-% |
| 200 - 250 µm | 11,8 Gew.-% |
| 150 - 200 µm | 4,7 Gew.-% |
| <150 µm | 0,5 Gew.-% |

Der Superabsorber wies eine mittlere Partikelgröße (d50) von 285 µm und eine mittlere Sphärizität (mSPHT) von 0,80 auf.

Herstellungsbeispiel 6 - Superabsorber B

Eingesetzt wurde ein Superabsorber vom Typ AQUA KEEP SA-60-SX-II (Sumitomo Seika Chemicals Co., Ltd.; Osaka; Japan). AQUA KEEP SA-60-SX-II ist ein durch umgekehrte Suspensionspolymerisation hergestellter Superabsorber.

Der Superabsorber hatte ein Schüttgewicht (ASG) von 0,66 g/ml, eine Zentrifugenretentionskapazität (CRC) von 34,0 g/g, eine Absorption unter einem Druck von 0,0 g/cm$^2$ (AUNL) von 54,8 g/g, eine Absorption unter einem Druck von 21,0 g/cm$^2$ (AUL) von 32,8 g/g, eine Absorption unter einem Druck von 49,2 g/cm$^2$ (AUHL) von 14,7 g/g, eine Flüssigkeitsweiterleitung (SFC) von $0 \times 10^{-7}$ cm$^3$s/g, einen Vortex von 46 s und eine Feuchtegehalt von 11,1 Gew.-%.

Der Superabsorber wies die folgende Partikelgrößenverteilung auf:

| | |
|---|---|
| >710 µm | 6,5 Gew.-% |
| 600 - 710 µm | 9,6 Gew.-% |
| 500 - 600 µm | 16,1 Gew.-% |
| 400 - 500 µm | 24,7 Gew.-% |
| 300 - 400 µm | 28,2 Gew.-% |
| 150 - 300 µm | 14,6 Gew.-% |
| 106 - 150 µm | 0,3 Gew.-% |
| <106 µm | 0,0 Gew.-% |

Der Superabsorber wies eine mittlere Partikelgröße (d50) von 429 µm und eine mittlere Sphärizität (mSPHT) von 0,68 auf.

Der Superabsorber A und der Superabsorber B wurden in unterschiedlichen Verhältnissen gemischt und die erhaltenen Superabsorbermischungen M wurde analysiert. Die Ergebnisse sind in den Tabellen 1 bis 6 zusammengefasst:

Tabelle 1: Mischungen aus Superabsorber A (Beispiel 3) und Superabsorber B (Beispiel 1)

| Beispiel | Superabsorber | Superabsorber | CRC | T20 | VAUL |
|---|---|---|---|---|---|
| | B | A | g/g | s | s |
| 1*) | 100% | 0% | 44,4 | > 1200 | 2567 |
| 2*) | 80% | 20% | 40,0 | > 1200 | 1330 |
| 3*) | 60% | 40% | 35,8 | > 1200 | 386 |
| 4*) | 40% | 60% | 33,0 | 393 | 243 |
| 5 | 20% | 80% | 28,5 | 210 | 179 |
| 6*) | 0% | 100% | 26,0 | 165 | 180 |
| *) Vergleichsbeispiel | | | | | |

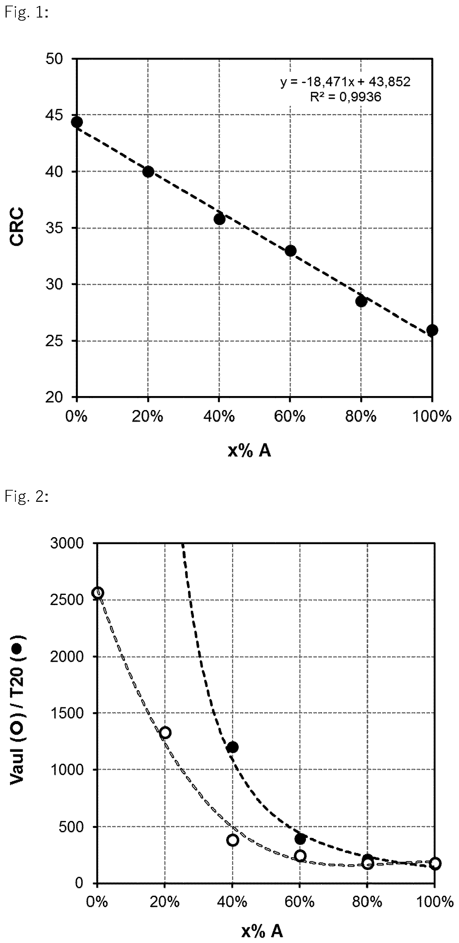

Die Ergebnisse der Tabelle 1 sind in den Figuren 1 und 2 dargestellt.

Tabelle 2: Mischungen aus Superabsorber A (Beispiel 5) und Superabsorber B (Beispiel 4)

| Beispiel | Superabsorber | Superabsorber | CRC | T20 | VAUL |
|---|---|---|---|---|---|
| | B | A | g/g | s | s |
| 7*) | 100% | 0% | 46,8 | > 1200 | 887 |
| 8*) | 80% | 20% | 44,3 | 749 | 762 |
| 9*) | 60% | 40% | 39,6 | 396 | 689 |
| 10*) | 40% | 60% | 36,5 | 300 | 470 |
| 11 | 20% | 80% | 31,6 | 220 | 365 |
| 12*) | 0% | 100% | 28,6 | 182 | 254 |
| *) Vergleichsbeispiel | | | | | |

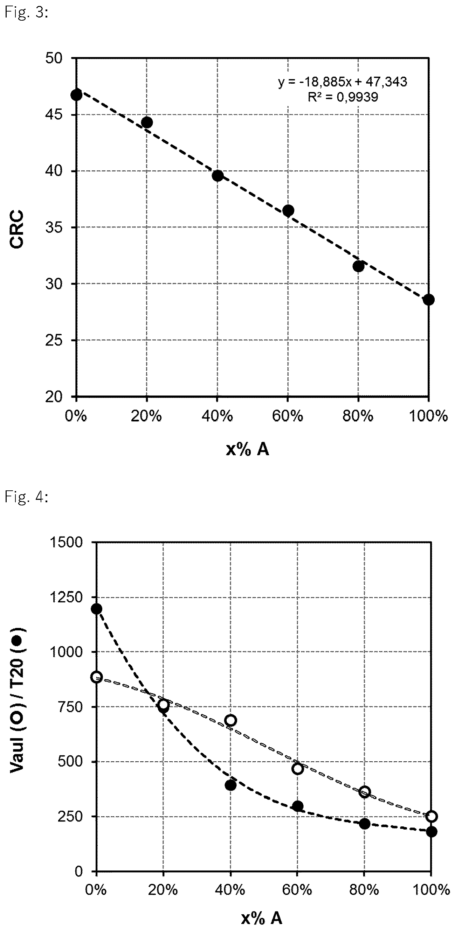

Die Ergebnisse der Tabelle 2 sind in den Figuren 3 und 4 dargestellt.

Tabelle 3: Mischungen aus Superabsorber A (Beispiel 3) und Superabsorber B (Beispiel 4)

| Beispiel | Superabsorber | Superabsorber | CRC | T20 | VAUL |
|---|---|---|---|---|---|
| | B | A | g/g | s | s |
| 13*) | 100% | 0% | 46,8 | > 1200 | 887 |
| 14*) | 80% | 20% | 41,8 | 563 | 614 |
| 15*) | 60% | 40% | 37,6 | 317 | 422 |
| 16*) | 40% | 60% | 32,8 | 228 | 303 |
| 17 | 20% | 80% | 28,5 | 187 | 206 |
| 18*) | 0% | 100% | 26,0 | 165 | 180 |
| *) Vergleichsbeispiel | | | | | |

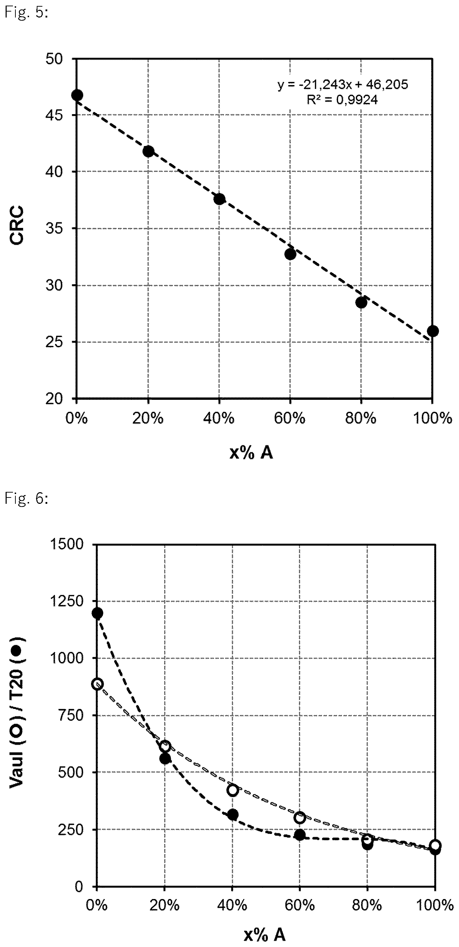

Die Ergebnisse der Tabelle 3 sind in den Figuren 5 und 6 dargestellt.

Tabelle 4: Mischungen aus Superabsorber A (Beispiel 5) und Superabsorber B (Beispiel 1)

| Beispiel | Superabsorber | Superabsorber | CRC | T20 | VAUL |
|---|---|---|---|---|---|
| | B | A | g/g | s | s |
| 19*) | 100% | 0% | 44,4 | > 1200 | 2567 |
| 20*) | 80% | 20% | 41,0 | > 1200 | 1309 |
| 21*) | 60% | 40% | 38,4 | > 1200 | 561 |
| 22*) | 40% | 60% | 34,0 | 546 | 378 |
| 23 | 20% | 80% | 30,7 | 323 | 300 |
| 24*) | 0% | 100% | 28,6 | 182 | 254 |
| *) Vergleichsbeispiel | | | | | |

Die Ergebnisse der Tabelle 4 sind in den Figuren 7 und 8 dargestellt.

Tabelle 5: Mischungen aus Superabsorber A (Beispiel 3) und Superabsorber B (Beispiel 6)

| Beispiel | Superabsorber | Superabsorber | CRC | T20 | VAUL |
|---|---|---|---|---|---|
| | B | A | g/g | s | s |
| 25*) | 100% | 0% | 34,0 | > 1200 | 1398 |
| 26*) | 80% | 20% | 32,1 | 753 | 520 |
| 27*) | 60% | 40% | 29,9 | 222 | 177 |
| 28*) | 40% | 60% | 28,0 | 167 | 145 |
| 29 | 20% | 80% | 26,9 | 155 | 161 |
| 30*) | 0% | 100% | 26,0 | 165 | 180 |
| *) Vergleichsbeispiel | | | | | |

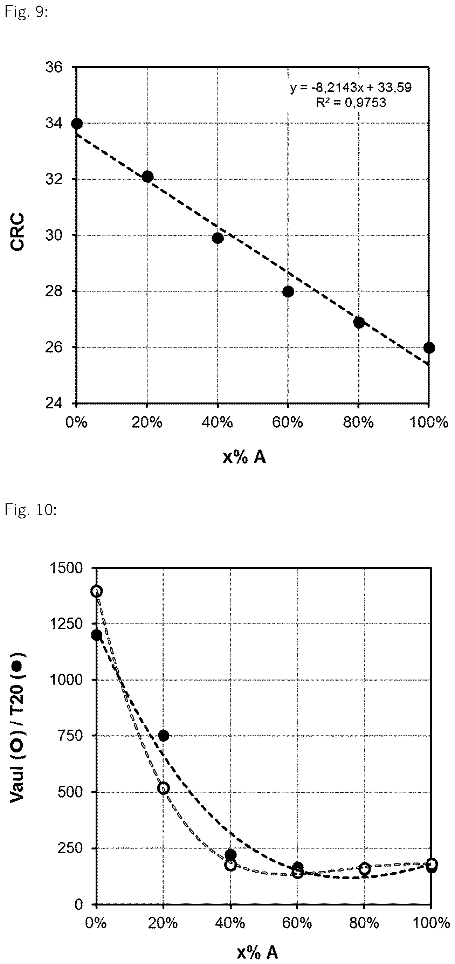

Die Ergebnisse der Tabelle 5 sind in den Figuren 9 und 10 dargestellt.

Tabelle 6: Mischungen aus Superabsorber A (Beispiel 3) und Superabsorber B (Beispiel 2)

| Beispiel | Superabsorber | Superabsorber | CRC | T20 | VAUL |
|---|---|---|---|---|---|
| | B | A | g/g | s | s |
| 31*) | 100% | 0% | 33,4 | 285 | **362** |
| 32*) | 80% | 20% | 32,6 | 271 | **323** |
| 33*) | 60% | 40% | 29,3 | 217 | **287** |
| 34*) | 40% | 60% | 27,6 | 182 | **192** |
| 35 | 20% | 80% | 26,1 | 143 | **163** |
| 36*) | 0% | 100% | 26,0 | 165 | **180** |
| *) Vergleichsbeispiel | | | | | |

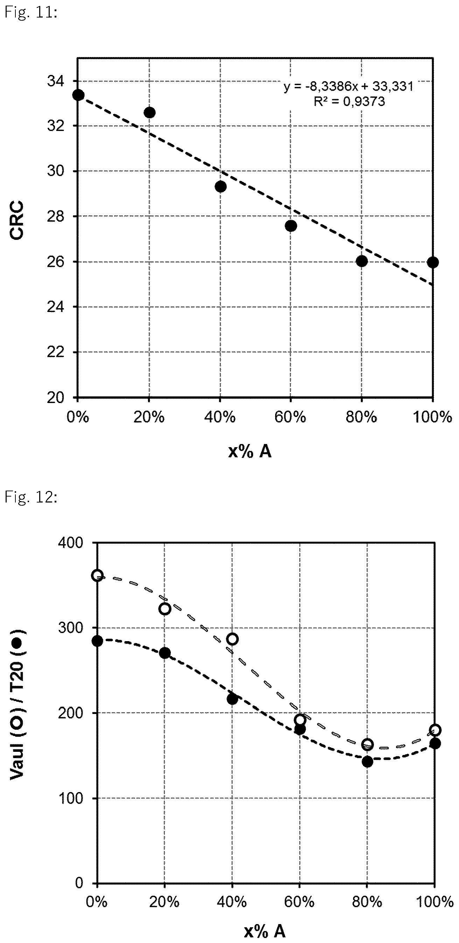

Die Ergebnisse der Tabelle 6 sind in den Figuren 11 und 12 dargestellt.

**Patentansprüche**

1.  Superabsorbermischung M, enthaltend mindestens 70 Gew.-% Superabsorber A mit einer Flüssigkeitsaufnahme von 20 g/g (T20) von weniger als 300 s und/oder einer volumetrischen Flüssigkeitsaufnahme unter 0,3 psi (2,07 kPa) Druck (VAUL) mit einem t-Wert von weniger als 400 s und mindestens 5 Gew.-% Superabsorber B mit einer Zentrifugenretentionskapazität (CRC) von mindestens 30 g/g, wobei die Flüssigkeitsaufnahme von 20 g/g (T20) gemäß der in der EP 2 535 027 A1 auf den Seiten 13 bis 18 beschriebenen Testmethode "K(t) Test Method (Dynamic Effective Permeability and Uptake Kinetics Measurement Test Method)" bestimmt wird, bei der volumetrischen Flüssigkeitsaufnahme unter 0,3 psi (2,07 kPa) Druck (VAUL) der t-Wert gemäß der in der WO 2014/079694 A1 auf den Seiten 39 und 40 beschriebenen Testmethode "Volumetrie Absorbency Under Load (VAUL)" bestimmt wird, wobei der t-Wert wird dort als "characteristic swelling time" bezeichnet wird, und die Zentrifugenretentionskapazität (CRC) gemäß der von der EDANA empfohlenen Testmethode Nr. WSP 241.2 (05) "Fluid Retention Capacity in Saline, After Centrifugation" bestimmt wird.

2.  Superabsorbermischung M nach Anspruch 1, wobei der Superabsorber A eine Flüssigkeitsaufnahme von 20 g/g (T20) von weniger als 240 s und/oder einer volumetrischen Flüssigkeitsaufnahme unter 0,3 psi (2,07 kPa) Druck (VAUL) mit einem t-Wert von weniger als 350 s aufweist.

3.  Superabsorbermischung M nach Anspruch 1 oder 2, wobei der Superabsorber A eine Flüssigkeitsaufnahme von 20 g/g (T20) von weniger als 180 s und/oder einer volumetrischen Flüssigkeitsaufnahme unter 0,3 psi (2,07 kPa) Druck (VAUL) mit einem t-Wert von weniger als 250 s aufweist.

4. Superabsorbermischung M nach einem der Ansprüche 1 bis 3, wobei der Superabsorber A eine Flüssigkeitsaufnahme von 20 g/g (T20) von weniger als 120 s und/oder einer volumetrischen Flüssigkeitsaufnahme unter 0,3 psi (2,07 kPa) Druck (VAUL) mit einem t-Wert von weniger als 200 s aufweist.

5. Superabsorbermischung M nach einem der Ansprüche 1 bis 4, wobei der Superabsorber B eine Zentrifugenretentionskapazität (CRC) von mindestens 35 g/g aufweist.

6. Superabsorbermischung M nach einem der Ansprüche 1 bis 5, wobei der Superabsorber B eine Zentrifugenretentionskapazität (CRC) von mindestens 40 g/g aufweist.

7. Superabsorbermischung M nach einem der Ansprüche 1 bis 6, wobei der Superabsorber B eine Zentrifugenretentionskapazität (CRC) von mindestens 45 g/g aufweist.

8. Superabsorbermischung M nach einem der Ansprüche 1 bis 7, wobei der Superabsorber A eine mittlere Sphärizität (mSPHT) von weniger als 0,72 aufweist, wobei die mittlere Sphärizität (mSPHT) mit dem PartAn® 3001 L Partikel Analysator (Microtrac Europe GmbH; Deutschland) bestimmt wird.

9. Superabsorbermischung M nach einem der Ansprüche 1 bis 8, wobei der Superabsorber B eine mittlere Sphärizität (mSPHT) von größer 0,72 aufweist.

10. Superabsorbermischung M nach einem der Ansprüche 1 bis 9, wobei der Superabsorber A ein Schüttgewicht (ASG) von weniger als 0,70 g/ml aufweist, wobei das Schüttgewicht (ASG) gemäß der von der EDANA empfohlenen Testmethode Nr. WSP 260.2 (05) "Density, Gravimetrie Determination" bestimmt wird.

11. Superabsorbermischung M nach einem der Ansprüche 1 bis 10, wobei der Superabsorber B ein Schüttgewicht (ASG) von größer 0,70 g/ml aufweist.

12. Superabsorbermischung M nach einem der Ansprüche 1 bis 11, wobei der Superabsorber A oberflächennachvernetzt ist.

13. Superabsorbermischung M nach einem der Ansprüche 1 bis 12, wobei der Superabsorber B nicht oberflächennachvernetzt ist.

14. Superabsorbermischung M nach einem der Ansprüche 1 bis 13, wobei der Superabsorber A und/oder der Superabsorber B eine mittlere Partikelgröße von 250 bis 500 μm aufweist, wobei die mittlere Partikelgröße mittels der von der EDANA empfohlenen Testmethode Nr. WSP 220.2 (05) "Partikel Size Distribution" ermittelt wird, wobei die Massenanteile der Siebfraktionen kumuliert aufgetragen werden und die mittlere Partikelgröße graphisch bestimmt wird und die mittlere Partikelgröße ist hierbei der Wert der Maschenweite, der sich für kumulierte 50 Gew.-% ergibt.

15. Hygieneartikel, enthaltend eine Superabsorbermischung M nach einem der Ansprüche 1 bis 14.

**Claims**

1. A superabsorbent mixture M comprising at least 70% by weight of superabsorbent A having a liquid absorption of 20 g/g (T20) of less than 300 s and/or a volumetric liquid absorption under a pressure of 0.3 psi (2.07 kPa) (VAUL) with a t value of less than 400 s, and at least 5% by weight of superabsorbent B having a centrifuge retention capacity (CRC) of at least 30 g/g, where liquid absorption of 20 g/g (T20) is determined by the "K(t) Test Method (Dynamic Effective Permeability and Uptake Kinetics Measurement Test Method)" described in EP 2 535 027 A1 on pages 13 to 18, the t value for volumetric liquid absorption under a pressure of 0.3 psi (2.07 kPa) (VAUL) is determined by the "Volumetric Absorbency Under Load (VAUL)" test method described in WO 2014/079694 A1 on pages 39 and 40, wherein the t value is described as "characteristic swelling time", and the centrifuge retention capacity (CRC) is determined by EDANA recommended test method No. WSP 241.2 (05) "Fluid Retention Capacity in Saline, After Centrifugation".

2. The superabsorbent mixture M according to claim 1, wherein superabsorbent A has a liquid absorption of 20 g/g (T20) of less than 240 s and/or a volumetric liquid absorption under a pressure of 0.3 psi (2.07 kPa) (VAUL) with a t value of less than 350 s.

3. The superabsorbent mixture M according to claim 1 or 2, wherein superabsorbent A has a liquid absorption of 20 g/g (T20) of less than 180 s and/or a volumetric liquid absorption under a pressure of 0.3 psi (2.07 kPa) (VAUL) with a t value of less than 250 s.

4. The superabsorbent mixture M according to any of claims 1 to 3, wherein superabsorbent A has a liquid absorption of 20 g/g (T20) of less than 120 s and/or a volumetric liquid absorption under a pressure of 0.3 psi (2.07 kPa) (VAUL) with a t value of less than 200 s.

5. The superabsorbent mixture M according to any of claims 1 to 4, wherein superabsorbent B has a centrifuge retention capacity (CRC) of at least 35 g/g.

6. The superabsorbent mixture M according to any of claims 1 to 5, wherein superabsorbent B has a centrifuge retention capacity (CRC) of at least 40 g/g.

7. The superabsorbent mixture M according to any of claims 1 to 6, wherein superabsorbent B has a centrifuge retention capacity (CRC) of at least 45 g/g.

8. The superabsorbent mixture M according to any of claims 1 to 7, wherein superabsorbent A has a mean sphericity (mSPHT) of less than 0.72, where the mean sphericity (mSPHT) is determined with the PartAn® 3001 L particle analyzer (Microtrac Europe GmbH; Germany).

9. The superabsorbent mixture M according to any of claims 1 to 8, wherein superabsorbent B has a mean sphericity (mSPHT) of greater than 0.72.

10. The superabsorbent mixture M according to any of claims 1 to 9, wherein superabsorbent A has a bulk density (ASG) of less than 0.70 g/ml, where the bulk density (ASG) is determined by EDANA recommended test method No. WSP 260.2 (05) "Density, Gravimetric Determination".

11. The superabsorbent mixture M according to any of claims 1 to 10, wherein superabsorbent B has a bulk density (ASG) of greater than 0.70 g/ml.

12. The superabsorbent mixture M according to any of claims 1 to 11, wherein superabsorbent A has been surface postcrosslinked.

13. The superabsorbent mixture M according to any of claims 1 to 12, wherein superabsorbent B has not been surface postcrosslinked.

14. The superabsorbent mixture M according to any of claims 1 to 13, wherein superabsorbent A and/or superabsorbent B have/has an average particle size of 250 to 500 $\mu$m, where the average particle size is determined by EDANA recommended test method No. WSP 220.2 (05) "Particle Size Distribution", by plotting the proportions by mass of the screen fractions in cumulated form and determining the average particle size from the graph, where the average particle size is the value of the mesh size which arises for a cumulative 50% by weight.

15. A hygiene article comprising a superabsorbent mixture M according to any of claims 1 to 14.

**Revendications**

1. Mélange de superabsorbants M, contenant au moins 70% en poids de superabsorbant A présentant une absorption de liquide de 20 g/g (T20) inférieure à 300 s et/ou une absorption volumétrique de liquide sous une pression de 0,3 psi (2,07 kPa) (VAUL) d'une valeur t inférieure à 400 s et au moins 5% en poids de superabsorbant B présentant une capacité de rétention à la centrifugeuse (CRC) d'au moins 30 g/g, l'absorption de liquide de 20 g/g (T20) étant déterminée selon la méthode de test décrite dans le document EP 2 535 027 A1 aux pages 13 à 18 « K(t) Test Method (Dynamic Effective Permeability and Uptake Kinetics Measurement Test Method) », lors de l'absorption volumétrique de liquide sous une pression de 0,3 psi (2,07 kPa) (VAUL), la valeur t étant déterminée selon la méthode de test décrite dans le document WO 2014/079694 A1 aux pages 39 et 40 « Volumétrie Absorbency Under Load (VAUL) », la valeur t y étant désignée par « characteristic swelling time » et la capacité de rétention à la centrifugeuse (CRC) étant déterminée selon la méthode de test recommandée par l'EDANA n° WSP 241.2 (05)

« Fluid Retention Capacity in Saline, After Centrifugation ».

2.  Mélange de superabsorbants M selon la revendication 1, le superabsorbant A présentant une absorption de liquide de 20 g/g (T20) inférieure à 240 s et/ou une absorption volumétrique de liquide sous une pression de 0,3 psi (2,07 kPa) (VAUL) d'une valeur t inférieure à 350 s.

3.  Mélange de superabsorbants M selon la revendication 1 ou 2, le superabsorbant A présentant une absorption de liquide de 20 g/g (T20) inférieure à 180 s et/ou une absorption volumétrique de liquide sous une pression de 0,3 psi (2,07 kPa) (VAUL) d'une valeur t inférieure à 250 s.

4.  Mélange de superabsorbants M selon l'une des revendications 1 à 3, le superabsorbant A présentant une absorption de liquide de 20 g/g (T20) inférieure à 120 s et/ou une absorption volumétrique de liquide sous une pression de 0,3 psi (2,07 kPa) (VAUL) d'une valeur t inférieure à 200 s.

5.  Mélange de superabsorbants M selon l'une des revendications 1 à 4, le superabsorbant B présentant une capacité de rétention à la centrifugeuse (CRC) d'au moins 35 g/g.

6.  Mélange de superabsorbants M selon l'une des revendications 1 à 5, le superabsorbant B présentant une capacité de rétention à la centrifugeuse (CRC) d'au moins 40 g/g.

7.  Mélange de superabsorbants M selon l'une des revendications 1 à 6, le superabsorbant B présentant une capacité de rétention à la centrifugeuse (CRC) d'au moins 45 g/g.

8.  Mélange de superabsorbants M selon l'une des revendications 1 à 7, le superabsorbant A présentant une sphéricité moyenne (mSPHT) inférieure à 0,72, la sphéricité moyenne (mSPHT) étant déterminée à l'aide de l'analyseur de particules PartAn® 3001 L (Microtrac Europe GmbH ; Allemagne).

9.  Mélange de superabsorbants M selon l'une des revendications 1 à 8, le superabsorbant B présentant une sphéricité moyenne (mSPHT) supérieure à 0,72.

10. Mélange de superabsorbants M selon l'une des revendications 1 à 9, le superabsorbant A présentant une masse volumique apparente (ASG) inférieure à 0,70 g/ml, la masse volumique apparente (ASG) étant déterminée selon la méthode de test recommandée par l'EDANA n° WSP 260.2 (05) « Density, Gravimétrie Détermination ».

11. Mélange de superabsorbants M selon l'une des revendications 1 à 10, le superabsorbant B présentant une masse volumique apparente (ASG) supérieure à 0,70 g/ml.

12. Mélange de superabsorbants M selon l'une des revendications 1 à 11, le superabsorbant A étant postréticulé en surface.

13. Mélange de superabsorbants M selon l'une des revendications 1 à 12, le superabsorbant B n'étant pas postréticulé en surface.

14. Mélange de superabsorbants M selon l'une des revendications 1 à 13, le superabsorbant A et/ou le superabsorbant B présentant une grosseur moyenne de particule de 250 à 500 um, la grosseur moyenne de particule étant déterminée selon la méthode de test recommandée par l'EDANA n° WSP 220.2 (05) "Partikel Size Distribution", les proportions massiques des fractions tamisées étant reportées de manière cumulée et la grosseur moyenne de particule étant déterminée graphiquement et la grosseur moyenne de particule étant la valeur de la largeur de maille obtenue pour 50% en poids cumulés.

15. Article hygiénique contenant un mélange de superabsorbants M selon l'une des revendications 1 à 14.

Fig. 1:

$$y = -18,471x + 43,852$$
$$R^2 = 0,9936$$

Fig. 2:

Fig. 3:

Fig. 4:

Fig. 5:

Fig. 6:

Fig. 7:

x% A

Fig. 8:

x% A

Fig. 9:

$y = -8,2143x + 33,59$
$R^2 = 0,9753$

Fig. 10:

Fig. 11:

Fig. 12:

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2014118024 A1
- US 20170361305 A
- WO 0031157 A1
- WO 9955767 A1
- EP 0530438 A1
- EP 0547847 A1
- EP 0559476 A1
- EP 0632068 A1
- WO 9321237 A1
- WO 03104299 A1
- WO 03104300 A1
- WO 03104301 A1
- DE 10331450 A1
- DE 10331456 A1
- DE 10355401 A1
- DE 19543368 A1
- DE 19646484 A1
- WO 9015830 A1
- WO 02032962 A2
- WO 2017170604 A1
- WO 2001038402 A1
- DE 3825366 A1
- US 6241928 B
- WO 2014079694 A1
- WO 2015110321 A1
- WO 2008068208 A1
- WO 2015062883 A2

- EP 0083022 A2
- EP 0543303 A1
- EP 0937736 A2
- DE 3314019 A1
- DE 3523617 A1
- EP 0450922 A2
- DE 10204938 A1
- US 6239230 B
- EP 0999938 A2
- DE 4020780 C1
- DE 19807502 A1
- DE 19807992 C1
- DE 19854573 A1
- DE 19854574 A1
- DE 10204937 A1
- EP 2204388 A1
- DE 10334584 A1
- EP 1199327 A2
- WO 03031482 A1
- DE 3713601 A1
- EP 0703265 B1
- US 5840321 A
- EP 2535027 A1
- EP 0640330 A1
- WO 2017207330 A1
- WO 2016134905 A1

**In der Beschreibung aufgeführte Nicht-Patentliteratur**

- **F.L. BUCHHOLZ ; A.T. GRAHAM.** Modern Superabsorbent Polymer Technology. Wiley-VCH, 1998, 71-103
- Standard Test Methods for the Nonwovens Industry. *Worldwide Strategie Partners,* 2005

- **EUGÈNE PLASKY.** EDANA. www.edana.org, vol. 157, 1030
- 1100 Crescent Green, Suite 115, Cary, North Carolina 27518, USA. INDA. www.inda.org